(19) Europäisches Patentamt
European Patent Office
Office européen des brevets

(11) Veröffentlichungsnummer: **0 190 096 B1**

(12) # EUROPÄISCHE PATENTSCHRIFT

(45) Veröffentlichungstag der Patentschrift: **16.08.89**

(51) Int. Cl.⁴: **A 01 N 53/00**, C 07 C 69/743, C 07 C 69/65

(21) Anmeldenummer: **86810002.5**

(22) Anmeldetag: **08.01.86**

(54) **Verfahren zum Schützen von keratinhaltigem Material gegen Befall durch Keratinschädlinge und neue Ester.**

(30) Priorität: **14.01.85 CH 150/85**

(43) Veröffentlichungstag der Anmeldung: **06.08.86 Patentblatt 86/32**

(45) Bekanntmachung des Hinweises auf die Patenterteilung: **16.08.89 Patentblatt 89/33**

(84) Benannte Vertragsstaaten: **BE CH DE FR GB LI**

(56) Entgegenhaltungen:
**FR-A-2 218 316**

**PESTICIDE SCIENCE, Band 13, Nr. 4, 1982, Seiten 407-414, Society of Chemical Industry; M. ELLIOTT et al.: "Insecticidal activity of the pyrethrins and related compounds. Part XII: alpha-substituted-3-phenoxybenzyl esters"**
**JOURNAL OF THE SOCIETY OF DYERS AND COLOURISTS, Band 98, Nr. 3, März 1982, Seiten 79-84, The Society of Dyers and Colourists and Contributors, Bradford, GB; B. DE SOUSA et al.: "The biological evaluation of pyrethroids as potential mothproofing agents. I - Esters of 2-substituted 3,3-dimethylcyclopropane-1-carboxylic acids"**
**JOURNAL OF THE SOCIETY OF DYERS AND COLOURISTS, Band 99, Nr. 2, Februar 1983, Seiten**

(73) Patentinhaber: **CIBA-GEIGY AG, Klybeckstrasse 141, CH-4002 Basel (CH)**

(72) Erfinder: **Farooq, Saleem, Dr., Kirchackerstrasse 27, CH-4422 Arisdorf (CH)**
Erfinder: **Reinehr, Dieter, Dr., Wolfsheul 10, D-7842 Kandern (DE)**
Erfinder: **Schmid, Werner, Keltenweg 40, CH-4125 Riehen (CH)**

(56) Entgegenhaltungen: (Fortsetzung)
**52-55, The Society of Dyers and Colourists and Contributors, Bradford, GB; B. DE SOUSA et al.: "The biological evaluation of pyrethroids as potential mothproofing agents. Part II - Esters of alpha-substituted phenylacetic acid"**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 190 096 B1

**Beschreibung**

In der GB-PS 1,413,491 der National Research Development Corporation sind Ester der Formel

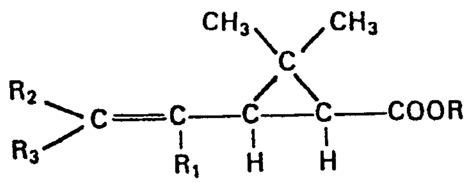

1

beschrieben, worin $R_1$ Wasserstoff oder einen Methylrest, $R_2$Wasserstoff, Halogen oder einen Alkylrest und $R_3$ Wasserstoff, Halogen oder eine Carbalkoxygruppe bedeuten, wobei R der Rest verschiedener Alkohole sein kann, darunter der 3-Phenoxybenzylrest. Bei den Estern der Formel (1) handelt es sich um Pyrethroide, die starke insektizide Eigenschaften aufweisen, von denen das wohl bekannteste Insektizid das Permethrin der Formel

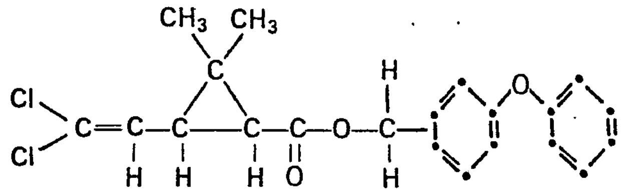

2

Es wurde nun gefunden, dass die insektizide Wirkung von bestimmten Estern der Formel (1), worin R den Rest eines 3-Phenoxybenzylalkohols darstellt, erheblich gesteigert werden kann, wenn statt des Wasserstoffs eine Phenyläthinylgruppe an die Methylengruppe des Alkohols gebunden ist. Solche Verbindungen eignen sich bei guter Pflanzenverträglichkeit und geringer Warmbluttoxizität sehr gut zum Bekämpfen von Schädlingen in Kulturpflanzen, insbesondere in Reiskulturen. Sie sind jedoch besonders zur Bekämpfung von keratinfressenden Käfern und deren Larven, vor allem Teppichkäferlarven, und daher zur Ausrüstung von keratinhaltigem Material gegen den Befall durch keratinfressende Insekten geeignet.

Gegenstand der Erfindung ist daher ein Verfahren zum Schützen bzw. Ausrüsten von Keratinmaterial bzw. keratinhaltigem Material vor bzw. gegen Befall durch keratinfressende Insekten, welches dadurch gekennzeichnet ist, dass man das zu schützende Material mit einem Ester der Formel

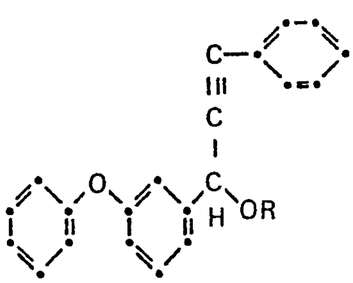

(3)

behandelt, worin R den Rest einer Säure der Formel

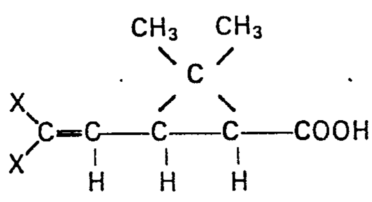

(4)

in der X einen Alkylrest mit 1–4 C-Atomen, Halogen oder eines der beiden X einen Phenylrest bedeutet, oder R den Rest der 4-Chlorphenylisopropyl-essigsäure darstellt.

Vorzugsweise behandelt man das zu schützende Material mit einem Ester der Formel

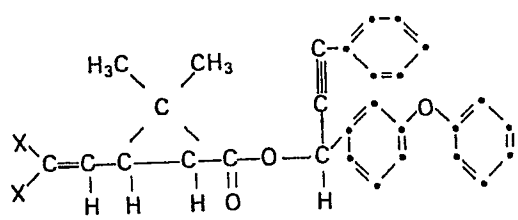

(5)

worin X einen Methylrest, Chlor oder Brom bedeutet, und insbesondere mit dem Ester der Formel

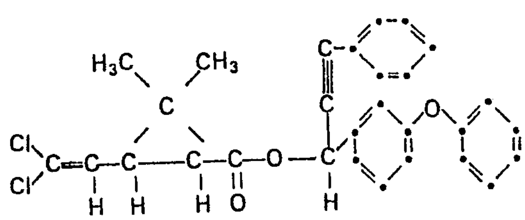

(6)

Bei dem Ester der Formel (3), worin R den Rest der 4-Chlorphenylisopropylessigsäure darstellt, und bei dem Ester der Formel (6) handelt es sich um bisher nicht bekannte und somit neue Verbindungen, während Verbindungen der Formel (5), worin X Brom oder Methyl bedeutet, bereits aus Pesticide Science, 1982, Seiten 407–414, bzw. FR-A 2, 218, 316 bekannt geworden sind. Diese beiden Verbindungen weisen nur eine geringe insektizide Wirkung bezüglich Keratinschädlingen auf.

Die Ester der Formel (3) können durch Umsetzung des Halogenids, insbesondere Chlorids, der 4-Chlorphenylisopropylessigsäure oder der Säure der Formel (4) mit 3-Phenoxy-α-phenyläthinylbenzylalkohol hergestellt werden. Der für die Umsetzung zu verwendende Alkohol kann durch Umsetzung von 3-Phenoxybenzaldehyd mit

Phenyläthinyl-magnesiumbromid erhalten werden.

Die zum Schützen von Keratinmaterial gegen keratinfressende Insekten zu verwendenden Ester wirken z.B. gegen keratinfressende Larven von Lepidoptera, z.B. Tineola spec. und Tinea spec., aber auch gegen keratinfressende Larven von Coleoptera, z.B. Anthrenus spec. und Attagenus spec. Sie eignen sich vorzüglich zum Schützen von Keratin-bzw. keratinhaltigem Material gegen Insektenfrass, insbesondere zur wasch- und lichtechten Ausrüstung gegen Insekten, insbesondere zur Motten- und Käferechtausrüstung von derartigen Materialien. Es kann Keratin- bzw. keratinhaltiges Material sowohl in rohem als auch in verarbeitetem Zustand ausgerüstet werden, z.B. rohe oder verarbeitete Schafwolle, Produkte aus anderen Tierhaaren, Felle, Pelze und Federn.

Praktisch besonders wichtig ist die Wirksamkeit der Verbindungen der Formel (3) gegen die Larven der Kleidermotte (Tineola. bisselliella), der Pelzmotte (Tinea pellionella) und der Samenmotte (Hofmannophila pseudospretella), aber auch gegen die Larven der Pelz- und Teppich-käfer (Attagenus spec. bzw. Anthrenus spec.), z.B. des Wollkraut- Blütenkäfers (Anthrenus verbasci), des Bibernell-Blütenkäfers (Anthrenus pimpinellae), des Gemeinen Teppichkäfers (Anthrenus scrophulariae), des Gefleckten Pelzkäfers (Attagenus pellio) und vor allem des Dunklen Pelzkäfers (Attagenus peceus) und des Teppichkäfers (Anthrenus flavipes).

Die bessere Käferschutzwirkung, speziell zu Teppichkäferlarven, als bei Permethrin, kann an folgenden Beispielen gezeigt werden:

1)Applikation nach dem Foulard-Verfahren

Teppichkäfer: Anthrenus flavipes; Gewichtsverlust in mg[1)]

| ppm AS[2)] appliziert | Ester Beispiel 1 | Permethrin |
|---|---|---|
| 30 | 33.4 | 129.5 |
| 60 | 11.9 | 92.8 |
| 125 | 6.5 | 47.8 |
| 250 | 1.5 | 4.1 |

2) Applikation nach dem Färbeverfahren

Teppichkäfer: Anthrenus flavipes; Gewichtsverlust in mg[1)]

| % AS[2)] appliziert | Ester Beispiel 1 | Permethrin |
|---|---|---|
| 0.0125 | 4.7 | 75.1 |
| 0.025 | 2.6 | 28.8 |
| 0.050 | 0.7 | 2.7 |
| 0.10 | 0.3 | 0.5 |

[1)] Biologische Prüfung nach SNV 195'901-1971 [2)] Aktivsubstanz

Bevorzugt wird das erfindungsgemässe Verfahren einerseits zum Schützen von Textilien aus Wolle, z.B. von Wolldecken, Wollteppichen, Wollwäsche, Wollkleidern und Wirkwaren bzw. wollhaltigen Textilien, wie Mischgeweben, deren eine Komponente Wolle ist, z.B. Mischgeweben aus Wolle und anderen Naturfasern, vorzugsweise Baumwolle oder aus Wolle und Kunstfasern, andererseits auch zum Schützen von Pelzen und Fellen vor dem Befall durch die erwähnten Schädlinge eingesetzt. Die Verbindungen der Formel (3) werden auf die oben erwähnten Substrate, insbesondere auf wollene und wollhaltige Textilien, vorzugsweise mit Hilfe der aus der Färbereipraxis bekannten Methoden wie Ausziehfärbeverfahren und Foulardapplikation aufgebracht. Zu diesem Zweck wird eine wässrige Lösung oder Dispersion (bzw. Emulsion oder Suspension) des jeweiligen Wirkstoffes angefertigt. Der Wirkstoff kann vorher in organischen Lösungsmitteln wie aliphatischen und alicyclischen Akloholen, Ketonen, Kohlenwasserstoffen wie Benzol, Xylolen, Toluol, Benzinen, ferner chlorierten und fluorierten Kohlenwasserstoffen, insbesondere in Propylenglykol, Methoxyäthanol, Äthoxyäthanol oder Dimethylformamid gelöst und anschliessend dem Applikationsbad zugegeben werden, das zusätzliche in der Färbereipraxis übliche Hilfsstoffe, z.B. Dispergatoren, Netzmittel, Säuren, Basen und/oder Farbstoffe enthalten kann, wobei derartige Hilfsstoffe bereits in der organischen Stammformulierung enthalten sein können.

Die Textilmaterialien können z.B. durch heisse oder kalte wässrige Färbe-, Bleich-, Chromierungs- oder Nachbehandlungsbäder mit den Wirkstoffen imprägniert werden, wobei verschiedene Textilausrüstungsverfahren, wie z.B. das Foulard- oder Ausziehverfahren, in Frage kommen.

Die Behandlung erfolgt zweckmässig bei Temperaturen von 10 bis 100 °C, im Färbebad vorzugsweise bei etwa 60–100 °C, um Nachbehandlungs- oder Waschbad bei vorzugsweise 10 bis 70, insbesondere 20 bis 60 °C.

Als zusätzliche Hilfsmittel können den Applikationsflotten z.B. Dispergatoren, Emulgatoren oder Tenside zugegeben werden. Daneben kann die Flotte auch noch übliche Hilfsstoffe, wie wasserlösliche Perborate, Polyphosphate, Carbonate, Silikate, optische Aufheller, Weichmacher, sauer reagierende Salze, wie Ammonium- oder Zinksilikonfluorid, oder gewisse organische Säuren, wie Oxalsäure, Essigsäure oder besonders Ameisensäure, ferner Antimikrobika und Appreturmittel, z.B. solche auf Kunstharzbasis oder Stärke, enthalten. Falls die Motten- und Käferechtausrüstung gemeinsam mit dem Färben des Materials (z.B. Wolle) durchgeführt wird, enthalten die Flotten auch noch die entsprechenden Farbstoffe und gegebenenfalls die dazu erforderlichen Hilfsmittel, z.B. Egalisiermittel.

Wässrige Applikationsflotten können z.B. Tenside, beispielsweise anionaktive Verbindungen, wie Seifen und andere Carboxylate (z.B. Alkalisalze höherer Fettsäuren), Abkömmlinge von Schwefel-Sauerstoffsäuren (z.B. Natriumsalz der Dodecylbenzolsulfonsäure, wasserlösliche Salze von Schwefelsäuremonoestern höhermolekularer Alkohole oder ihrer Polyglykoläther, wie etwa lösliche Salze von Dodecylalkoholsulfat oder von Didecylalkoholpolyglykoläther-sulfat), Abkömmlinge von Phosphor-Sauerstoffsäuren(z.B. Phosphate), Abkömmlinge mit saurem (elektrophilem) Stickstoff in der hydrophilen Gruppe (z.B. Disulfinsalze), kationaktive Tenside, wie Amine und ihre Salze (z.B. Lauryl-diäthylentriamin), Oniumverbindungen, Aminoxide oder nichtionogene Tenside, wie Polyhydroxyverbindungen, Tenside auf Mono- oder Polysaccharidbasis, höhermolekulare Acetylenglykole, Polyglykoläther (z.B. Polyglykoläther höherer Fettalkohole, Polyglykoläther höhermolekular-alkylierter Phenole) enthalten.

Im Falle von nicht-wässriger Applikation (Lösungsmittelapplikation) kann ein entsprechender Teil einer Verbindung der Formel (3) auch einem geeigneten Lösungsmittel zugegeben werden und mit der so erhaltenen Lösung kann das zu schützende Material imprägniert werden.

Als Lösungsmittel kommen hierfür unter anderen Trichloräthylen, Methylenchlorid, Kohlenwasserstoffe, Propylenglykol, Methoxyäthanol, Äthoxyäthanol, Dimethylformamid in Frage, denen noch Verteilungsmittel (z.B. Emulgatoren, wie sulfiertes Ricinusöl, Fettalkoholsulfate usw.) und/oder andere Hilfsstoffe zugesetzt werden können. Die zu schützenden Materialien werden üblicherweise mit diesen Lösungen einfach imprägniert.

Die Ausrüstung der zu schützenden Materialien kann aber auch mit einem Trockenreinigungsprozess kombiniert werden. Ein entsprechender Teil einer Verbindung der Formel (3) wird zu diesem Zweck im Reinigungsmittel (etwa niedere halogenierte Alkane, z.B. Trichloräthylen usw.) gelöst und der Reinigungsprozess wird wie üblich durchgeführt.

Ein Anteil einer Verbindung der Formel (3) kann aber auch in leicht flüchtigen organischen Lösungsmitteln gelöst werden und diese Lösung kann dann auf das zu schützende Substrat aufgesprüht werden (Sprühapplikation). Für diese Applikationsart eignen sich vor allem wollhaltige Textilien, Pelze und Federn. Der Vorteil der Sprühapplikation besteht darin, dass wegen der Rückgewinnung der Lösungsmittel eine Belastung der Abwässer vermieden wird.

Im erfindungsgemässen Verfahren können die Verbindungen der Formel (3) auch in Kombination mit anderen gegen keratinfressende Insekten wirksamen Schutzmitteln angewendet werden, z.B. in Kombination mit Harnstoffderivaten, Benzimidazolen, aromatischen Sulfonamiden, Phosphor- und Phosphonsäureestern und 5-Phenylcarbamoylbarbitursäurederivaten.

Die jeweils eingesetzte Menge an Verbindung der Formel (3), die in das jeweilige Applikationsbad bzw. das nichtwässrige Lösungsmittel eingebracht wird, hängt vom jeweiligen Substrat und der Applikationsmethode ab. Diese Menge wird üblicherweise jedoch so bemessen, dass nach dem Aufziehen auf das jeweils zu schützende Material letzteres etwa 10 bis 2 000 ppm, vorzugsweise 100 bis 1 000 ppm an Verbindung der Formel (3) enthält, wobei die obere Grenze weitgehend durch Überlegungen ökonomischer Natur gegeben ist, während die untere Grenze von Kriterien wie angestrebte Breite und Dauerhaftigkeit der Schutzwirkung abhängt. Dies ergibt z.B. für das Ausziehverfahren vei einem Flottenverhältnis von 1:20 Konzentrationen von 0,001 bis 1 g Wirksubstanz 1 Behandlungsbad, je nach erreichbarem Ausziehgrad. Beim Foulardverfahren sind Konzentrationen bis 2 g Wirksubstanz pro Liter möglich.

Die vorliegende Erfindung betrifft schliesslich auch Mittel zum Schützen bzw. Ausrüsten von Keratinmaterial bzw. keratinhaltigem Material gegen den Befall durch Keratinschädlinge, die die neuen Ester der Formel (3) enthalten. Die erfindungsgemässen Mittel können neben dem Wirkstoff beispielsweise auch noch übliche Träger und/oder Formulierungshilfsmittel, beispielsweise organische Läsungsmittel, Wasser, Säuren, Basen, Netz-, Dispergier- und/oder Emulgiermittel enthalten. Die erfindungsgemässen Mittel können auch noch Hilfsmittel enthalten, die weiter oben als Hilfsmittel in Applikationsflotten fur das erfindungsgemässe Ausrüstungsverfahren beschrieben sind. Ferner können die erfindungsgemässen Mittel auch noch andere gegen keratinfressende Insekten wirksame Schutzmittel enthalten, beispielsweise Harnstoffderivate, Benzimidazole, aromatische Sulfonamide, Phosphor- und Phosphonsäureester und/oder 5-Phenylcarbamoylbarbitursäurederivate.

Nachfolgend wird die Herstellung des 2,2-Dimethyl-3-(2,2-dichlorvinyl)cyclopropancarbonsäure-3-phenoxy-α-(2-phenyläthinyl)-benzylesters sowie des als Ausgangsverbindung verwendeten 3-Phenoxy-α -phenyläthinylbenzylalkohols beschrieben.

Beispiel 1

Zu 4,51 g 2,2-Dimethyl-3-(2,2-dichlorvinyl)-cyclopropylcarbonsäurechlorid, gelöst in 50 ml Toluol, werden unter Eiskühlung und Luftausschluss ($N_2$ -Spülung) 1,8 ml Pyridin und dann 6 g 3-Phenoxy-α-phenyläthinyl-benzylalkohol, gelöst in 20 ml Toluol, zugetropft. Die Reaktionsmischung wird über Nacht gerührt und dann mit je etwa 200 ml Toluol und Wasser verdünnt, nacheinander mit 2 n HC1, 10%-iger $K_2CO_3$-, 10%-iger $NaHCO_3$- und gesättigter Kochsalzlösung extrahiert, üger Natriumsulfat getrocknet und am Rotationsverdampfer eingeengt. Die eingeengte Lösung wird über 200 g Kieselgel filtriert und mit Hexan/Äther 95:5 eluiert. Nach Entfernung des Lösungsmittels erhält man 8,0 g 2,2-Dimethyl-3-(2,2-dichlorvinyl)-cyclopropan-

carbonsäure -3-Phenoxy- α- (2-phenyläthinyl)-benzylester der Formel

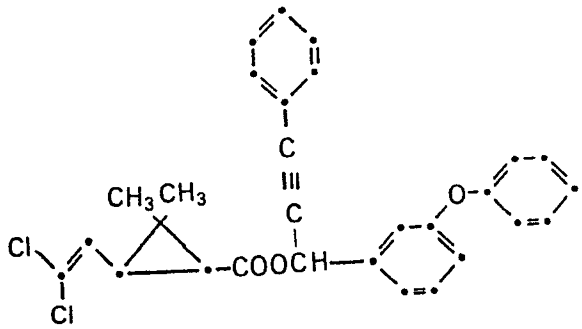

in Form eines hellgelben Öls mit einem Brechungsindex $n_D^{20} = 1{,}5943$ und einem in Fig. 1 dargestellten NMR-Spektrum. Die Ausbeute beträgt 81,8% der Theorie.

Beispiel 2

Der für das Pyrethroid benötigte Alkohol wird wie folgt hergestellt:

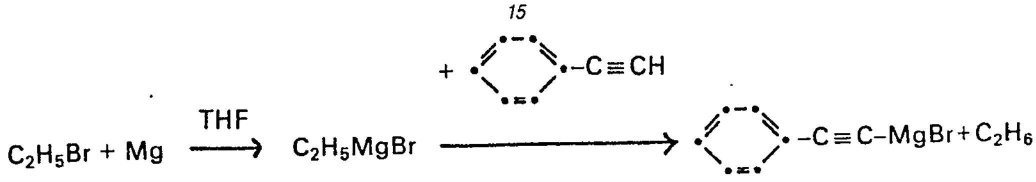


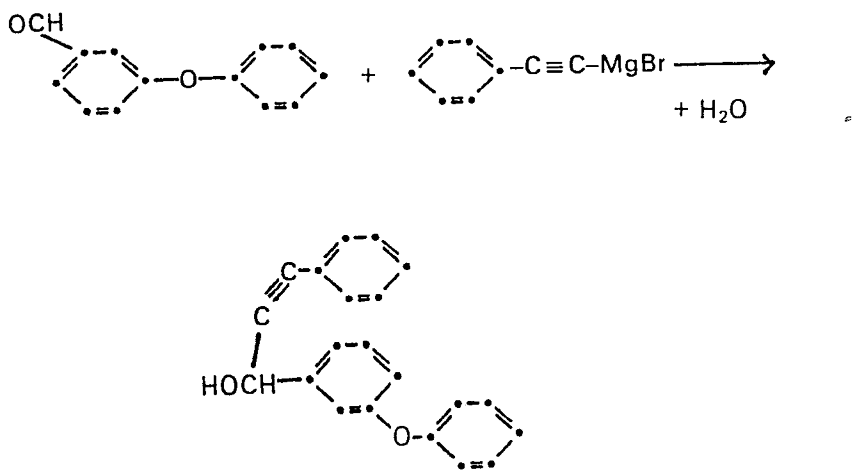

14,6 g Magnesiumspäne werden zusammen mit einer Spatelspitze an Jodkristallen vorgelegt. Darauf werden 73,6 g Äthylbromid in 25 ml THF (Tetrahydrofuran) so zugetropft, dass das THF leicht unter Rückfluss siedet. Die gebildete Grignardlösung wird bei 0-5 °C zu einer Lösung von 62,1 g Phenylacetylen in 250 ml THF zugetropft und solange gerührt, bis die Gasentwicklung aufhört. Die entstandene Lösung wird auf 0 °C gekühlt und tropfenweise mit 99 g 3-Phenoxybenzaldehyd so versetzt, dass die Temperatur nicht +5 °C übersteigt. Es wird über Nacht bei Raumtemperatur gerührt, auf 0 °C bis +5 °C gekühlt und mit ca. 100 g Eis versetzt, wobei sich ein Niederschlag bildet, der mit 100 ml konz. HC1 aufgelöst wird. Die wässrige Lösung wird dreimal mit je 150 ml Äther extrahiert, die Extrakte mit $NaHCO_3$-Lösung gewaschen und dann mit $Na_2SO_4$ getrocknet. Der Äther wird am Rotationsverdampfer abdestilliert. Man erhält als Rückstand 148 g 3-Phenoxy-α-phenyläthinyl-benzylalkohol mit einem Brechungsindex $n_D^{23} = 1{,}6238$ und einem in Fig. 2 dargestellten NMR-Spektrum. Die Ausbeute beträgt 98,8% der Theorie.

## Patentansprüche

1. Verfahren zum Schützen bzw. Ausrüsten von Keratinmaterial bzw. keratinhaltigem Material vor bzw. gegen Befall durch keratinfressende Insekten, dadurch gekennzeichnet, dass man das zu schützende Material mit einem Ester der Formel

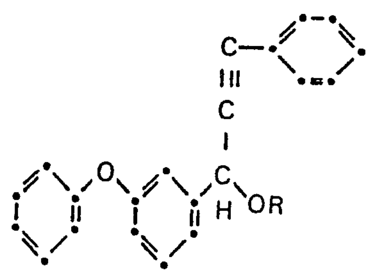

behandelt, worin R den Rest einer Säure der Formel

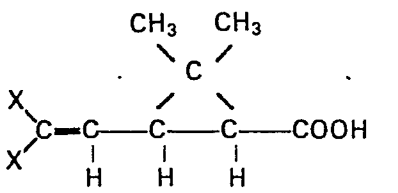

in der X einen Alkylrest mit 1-4 C-Atomen, Halogen oder eines der beiden X einen Phenylrest bedeutet, oder R den Rest der 4-Chlorphenylisopropylessigsäure darstellt.

2. Verfahren gemäss Anspruch 1, dadurch gekennzeichnet, dass man das zu schützende Material mit einem Ester der Formel

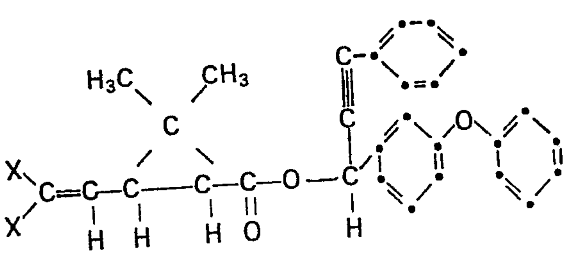

behandelt, worin X einen Mathylrest, Chlor oder Brom bedeutet.

3. Verfahren gemäss Anspruch 1, dadurch gekennzeichnet, dass man das zu schützende Material mit dem Ester der Formel

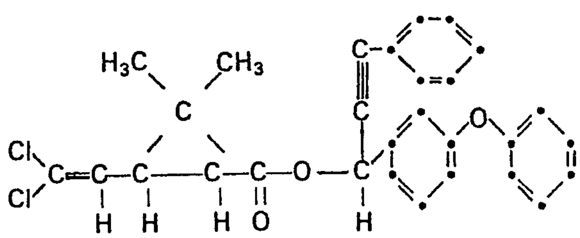

behandelt.

4. Ester der Formel

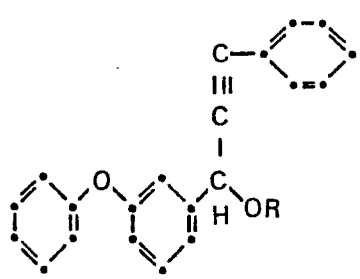

worin R den Rest der Säure der Formel

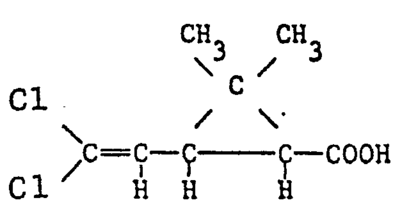

oder der 4-Chlorphenylisopropylessigsäure darstellt.

5. Ester gemäss Anspruch 4, gekennzeichnet durch die Formel

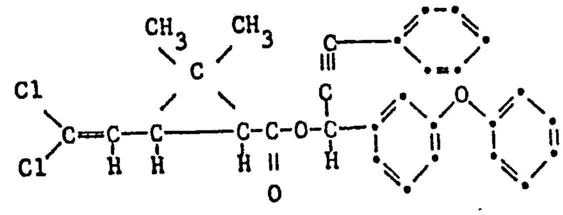

6. Ester gemäss Anspruch 4, gekennzeichnet durch die Formel

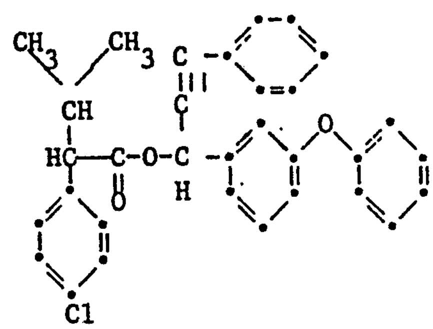

7. Das nach dem Verfahren gemäss Anspruch 1 behandelte Keratin- bzw. Keratinhaltige Material.

## Revendications

1. Procédé pour protéger ou apprêter la kératine ou les matières kératiniques contre l'attaque par les insectes dévorant la kératine, caractérisé en ce que l'on traite la matière à protéger par un ester de formule

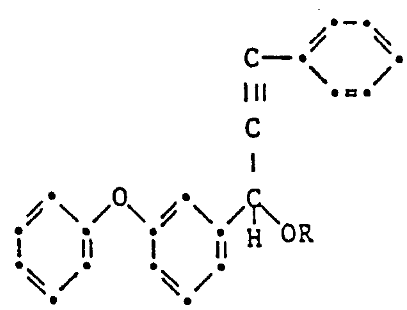

dans laquelle R est le radical d'un acide de formule

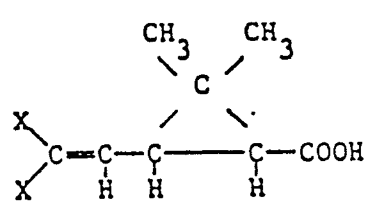

dans laquelle X représente un groupe alkyle en C 1-C 4, un halogène, ou bien l'un des deux symboles X représente un groupe phényle, ou bien R est le radical de l'acide 4-chlorophénylisopropylacétique.

2. Procédé selon la revendication 1, caractérisé

en ce que l'on traite la matière à protéger par un ester de formule

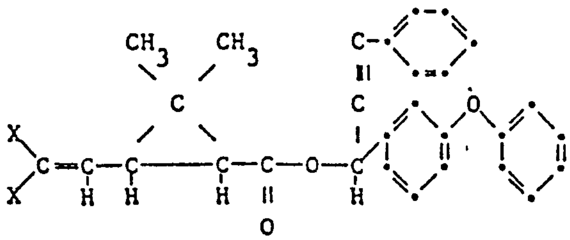

dans laquelle X représente un groupe méthyle, le chlore ou le brome.

3. Procédé selon la revendication 1, caractérisé en ce que l'on traite la matière à protéger par l'ester de formule

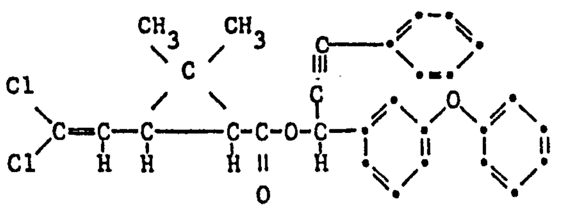

4. Esters de formule

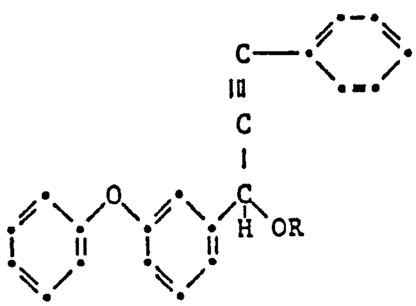

dans laquelle R représente le radical de l'acide de formule

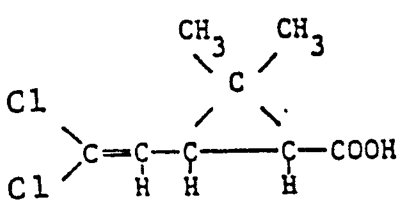

ou le radical de l'acide 4-chlorophénylisopropylacétique.

5. Ester selon la revendication 4, caractérisé par la formule

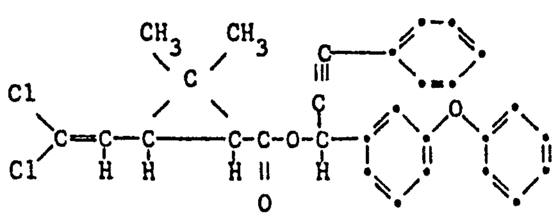

6. Ester selon la revendication 4, caractérisé par la formule

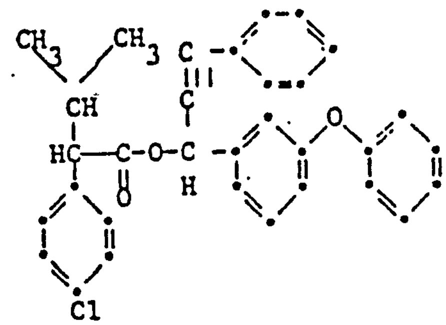

7. La kératine ou une matière kératinique traitée par le procédé selon la revendication 1.

## Claims

1. A process for protecting or proofing keratin material and keratin-containing material from or against attack by insects that feed on keratin, which process comprises treating the material to be protected with an ester of the formula

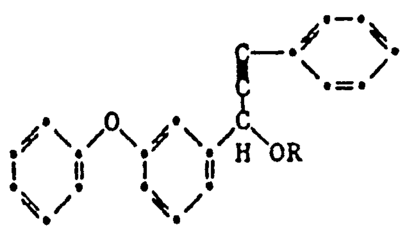

wherein R is the radical of an acid of the formula

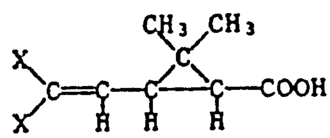

in which X is a $C_1$–$C_4$ alkyl group or halogen or one of the two substituents X is a phenyl group, or R is the radical of 4-chlorophenylisopropylacetic acid.

2. A process according to claim 1, which comprises treating the material to be protected with an ester of the formula

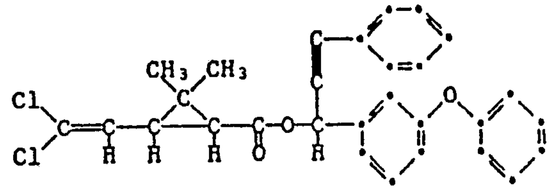

wherein X is a methyl group, chlorine or bromine.

3. A process according to claim 1, which comprises treating the material to be protected with an ester of the formula

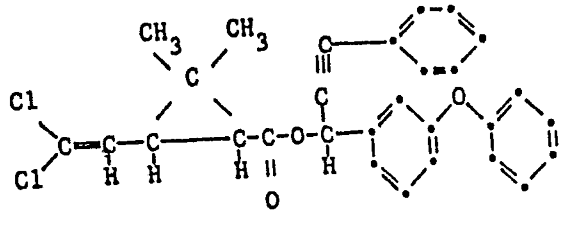

4. Esters of the formula

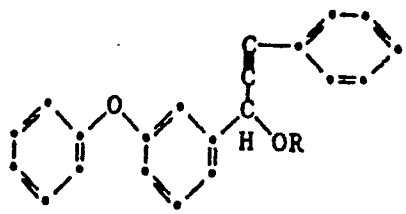

wherein R is the radical of an acid of the formula

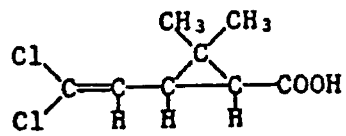

or of 4-chlorophenylisopropylacetic acid.

5. Esters according to claim 4 of the formula

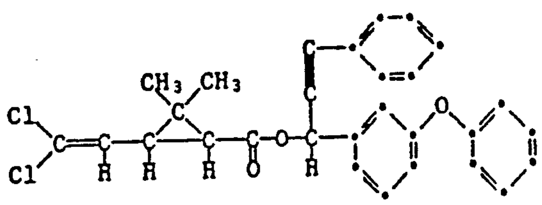

6. Esters according to claim 4 of the formula

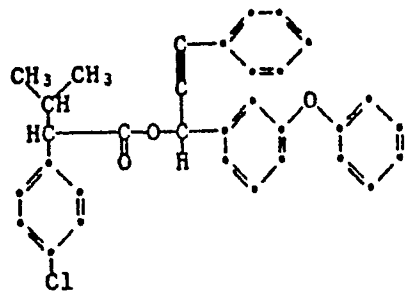

7. The keratin or keratin-containing material treated in accordance with the process of claim 1.

1/2

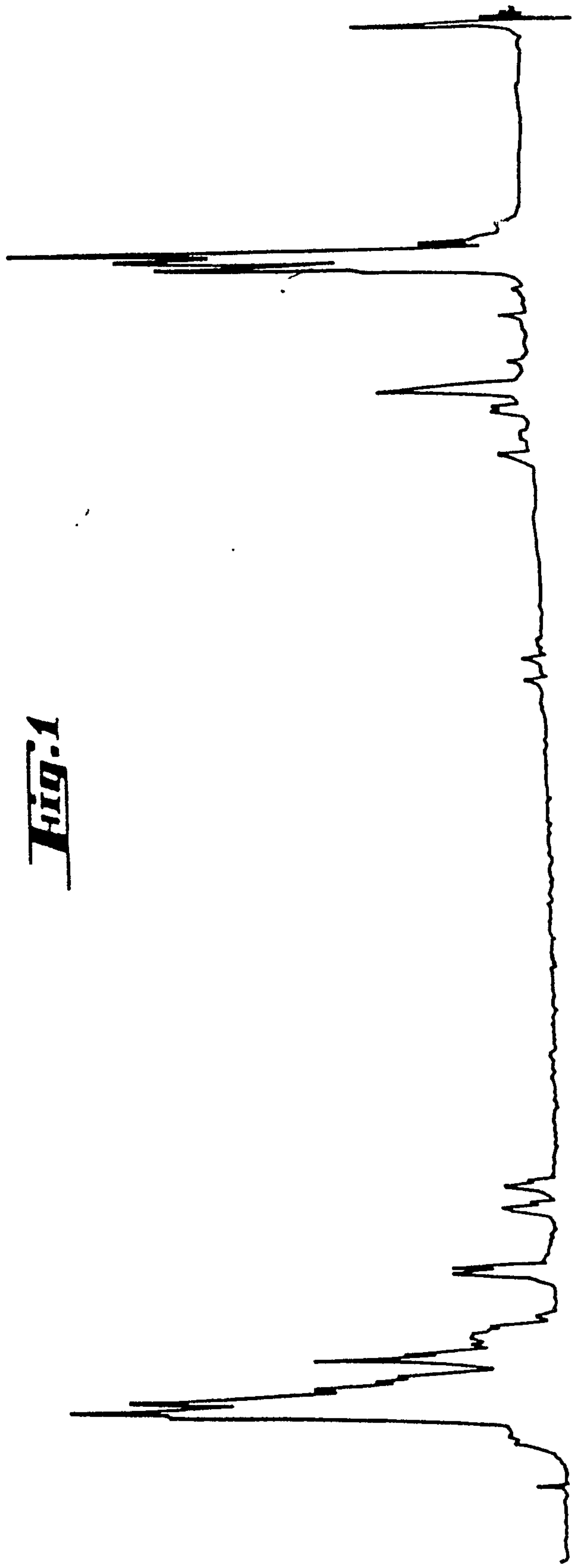

Fig.1

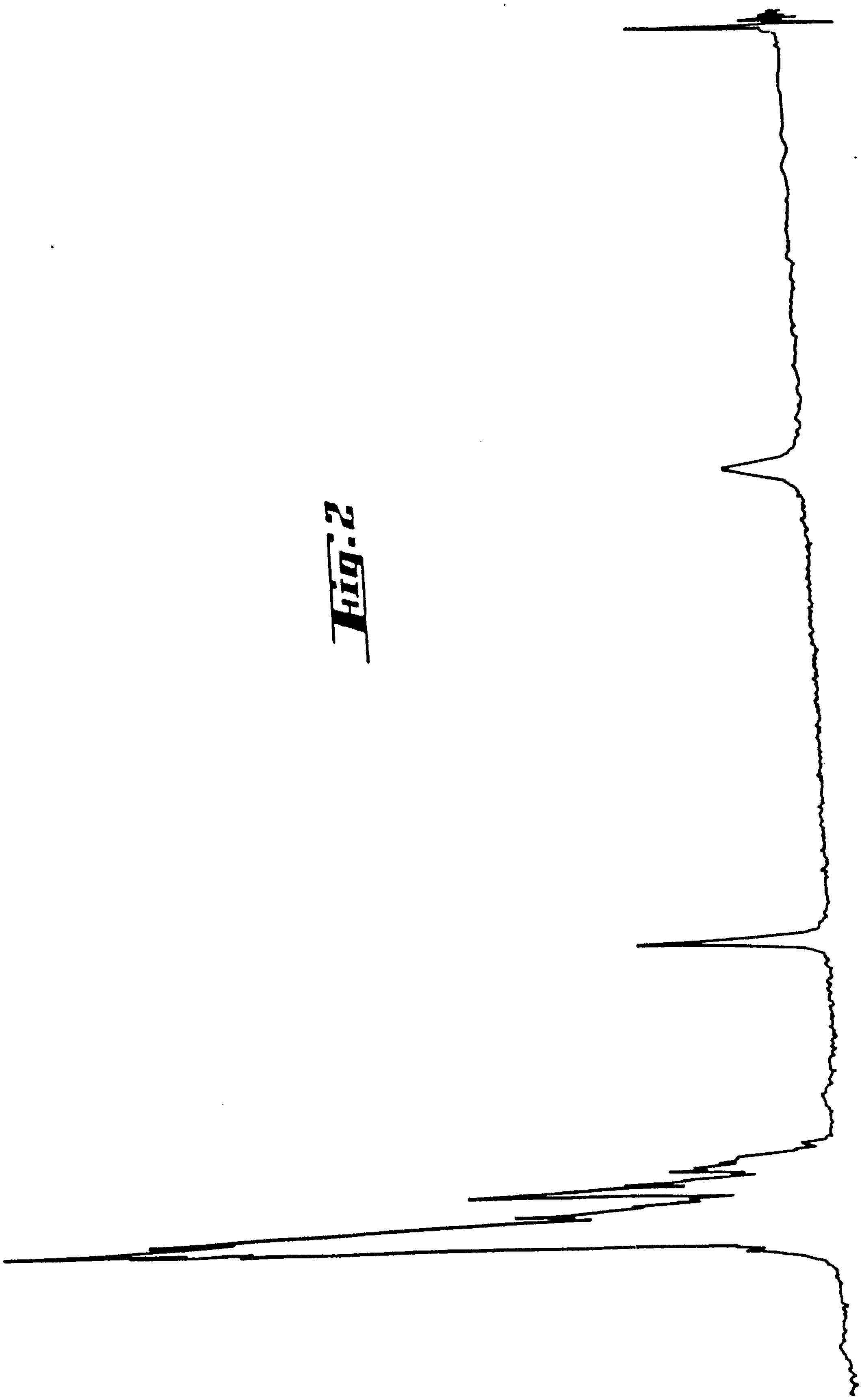

Fig. 2